# EUROPEAN PATENT SPECIFICATION

(11) **EP 1 365 643 B1**
(45) Date of publication and mention of the grant of the patent: **06.08.2008**
(21) Application number: 02718905.9
(22) Date of filing: 06.02.2002
(51) Int. Cl.: C12N 15/89, C12M 3/00, A01K 45/00, A01K 67/027

(54) **MICROINJECTION ASSEMBLY AND METHODS FOR MICROINJECTING AND REIMPLANTING AVIAN EGGS**
"MIKROINJEKTIONSAPPARAT, SOWIE VERFAHREN FÜR DIE MIKROINJEKTION UND REIMPLANTATION VON VOGELEIERN"
PROCEDES ET ASSEMBLAGE DE MICRO-INJECTION DESTINES A MICRO-INJECTER ET A REIMPLANTER DES OVOCYTES

(30) Priority: 13.02.2001 US 269012 P; 31.07.2001 US 919143
(43) Date of publication of application: 03.12.2003
(73) Proprietor: Avigenics, Inc., Athens, GA 30605 (US)
(72) Inventor: CHRISTMANN, Leandro, Watkinsville, GA 30677 (US)
(74) Representative: Silveston, Judith
(86) International application number: PCT/US2002/003302
(87) International publication number: WO 2002/064727

(56) References cited:
- WO-A-03/025146
- US-A- 4 907 158
- US-A- 5 225 750
- US-A- 5 784 992
- NAITO M ET AL: MOLECULAR REPRODUCTION AND DEVELOPMENT, vol. 37, 1994, pages 167-171, XP002964692
- LOVE J ET AL: BIO/TECHNOLOGY, vol. 12, 12 January 1994 (1994-01-12), pages 60-63, XP002918749
- PAIN B ET AL: "CHICKEN EMBRYONIC STEM CELLS AND TRANSGENIC STRATEGIES" , CELLS TISSUES ORGANS, KARGER, BASEL, CH, VOL. 165, PAGE(S) 212-219 XP000882175 ISSN: 1422-6405 * abstract * * page 213, column 2, paragraph 3 - page 214, column 1, paragraph 1 *
- IVARIE R: TRENDS IN BIOTECHNOLOGY, vol. 21, no. 1, January 2003 (2003-01), pages 14-19, XP004397632

## Description

### Field of the Invention

The present invention relates generally to an assembly for the microinjection of exogenous nucleic acid into avian embryonic cells or cytoplasts. More specifically, the present invention relates to a microscope and micromanipulator assembly for microinjecting an avian germinal disk on an opaque yolk. The present invention further relates to methods of microinjecting avian ova with exogenous nucleic acid, reimplanting the transgenic ova into a hen for laying, and development of the ova to viable chicks.

### Background

The field of animal transgenics, initially developed to understand the action of a single gene in the context of the whole animal and the phenomena of gene activation, is amongst the most powerful tools available for the study of genetics, and the understanding of genetic mechanisms and function.

Of equal importance, particularly from an economic perspective, is the use of transgenic technology to introduce heterologous DNA into animals of commercial importance to convert the animals into "factories" for the production of specific proteins or other substances of pharmaceutical interest (Gordon et al, 1987, Biotechnology 5: 1183-1187; Wilmut et al., 1990, Theriogenology 33: 113-123). Transgenic animals can express an exogenous protein, such as an antibody, under conditions that offer high yield of the protein in an active form and incorporating post-translational modifications such as glycosylation that are necessary for full functionality.

Historically, transgenic animals have been produced almost exclusively by microinjection of a fertilized egg. The pronuclei of fertilized eggs are microinjected *in vitro* with foreign, i.e., xenogeneic or allogeneic heterologous DNA or hybrid DNA molecules. The microinjected fertilized eggs are then transferred to the genital tract of a pseudopregnant female (e.g., Krimpenfort et al., in U.S. Pat. Nos. 5,175,384, 5,434,340 and 5,591,669).

This widely used technique requires large numbers of fertilized eggs, equipment to handle embryos and the facility to microinject them *in vitro.* There is a high rate of egg loss due to lysis during microinjection. Manipulated embryos are also less likely to implant and survive *in utero.* These factors contribute to the technique's extremely low efficiency. Consequently, generating large animals with these techniques is prohibitively expensive. Genetic information also has been transferred to embryos using retroviral vectors (Jaenisch, R., 1976, Proc. Natl. Acad. Sci. USA 73: 1260-1264), but the animals produced were mosaics with different gene insertions in different tissues. (Jaenisch, R., 1980, Cell 19: 181-188).

An alternative method is nuclear replacement of fertilized ova or Metaphase II-arrested eggs. Diploid cells are transfected *in vitro* by techniques commonly known in the art. The transfected diploid nuclei are isolated by micromanipulation and then transferred into an unactivated or activated egg, after which the "native" nuclear material is removed by suction. The egg cell then proceeds with embryonic development based on the transfected diploid nucleus. However, extreme skill is required for the technique of micromanipulation; therefore, the technique is costly and has a low success rate.

One system that holds potential as a protein bioreactor is the avian reproductive system. The production of an egg begins with formation of the large yolk in the ovary of the hen. The unfertilized oocyte or ovum is positioned on top of the yolk sac. Upon ovulation or release of the yolk from the ovary, it passes into the infundibulum of the oviduct where it is fertilized if sperm are present, and then moves into the magnum of the oviduct that is lined with tubular gland cells. These cells secrete the egg white proteins, including ovalbumin, lysozyme, ovomucoid, conalbumin and ovomucin, into the lumen of the magnum from which they are deposited onto the avian embryo and yolk.

The hen oviduct offers outstanding potential as a protein bioreactor because of high levels of protein production, the promise of proper folding and post-translation modification of the target protein, the ease of product recovery and the shorter developmental period of chickens compared to other potential transgenic species. Bosselman et al., in U.S. Patent No. 5,162,215, describe a method for introducing a replication-defective retroviral vector into a pluripotent stem cell of an unincubated chick embryo, and further describe chimeric chickens, whose cells express a heterologous vector nucleic acid sequence. However, the percentage of G1 transgenic offspring (progeny from vector-positive male G0 birds) was low and varied between 1% and approximately 8%.

Generally, direct DNA injection into avian eggs has led to poor and unstable transgene integration (Sang and Perry, 1989, Mol. Reprod. Dev. 1: 98-106 and Naito et al., 1994, Reprod. Dev. 37, 167-71. In addition, the use of viral vectors poses a number of limitations, including limited transgene size and potential viral infection of the offspring. The production of transgenic chickens by means of DNA microinjection (*supra*) has been both inefficient and time consuming. Ovum transfer, the transfer of a donor ovum to the oviduct of a recipient hen, provides another means for genetic manipulation in avians.

PCT Publication WO 87/05325 discloses a method of transferring material into sperm or egg cells by using liposomes. Bachiller et al. (1991, Mol. Reprod. Develop. 30, 194-200) uses Lipofectin-based liposomes to transfer DNA into mice sperm nuclei. Nakanishi and Iritani (1993, Mol. Reprod Develop. 36: 258-261) used Lipofectin-based liposomes to associate heterologous DNA with chicken sperm, which were in turn used to artificially inseminate hens. Tanaka et al. (1994, J. of Reprod. and Fertility 100: 447-449) produced chicks by *in vitro* fertilization (IVF) and then returning the fertilized ova to the oviduct of recipient hens to complete the egg and shell formation.

Another method for integrating heterologous DNA into avian sperm (Shemesh *et al.,* PCT International Publication WO 99/42569) is restriction enzyme mediated integration (REMI), which utilizes a linear DNA derived by cutting a plasmid with a restriction enzyme that generates single-stranded cohesive ends. The linear, cohesive-ended DNA, together with the restriction enzyme used to produce the cohesive ends is introduced into the target cells by electroporation. The restriction enzyme cuts the genomic DNA and enables the heterologous DNA to integrate via its matching cohesive ends (Schiestl and Petes, 1991, Proc. Natl. Acad. Sci. U.S.A. 88: 7585-7589).

US 5,225,750 discloses a microinjection apparatus comprising a micromanipulator for moving, by pressure, a liquid through a miniature instrument, comprising a microsyringe having a plunger and a reversible driving means for driving said plunger, where the driving means comprises a driven member on one end of said plunger with a piezoelectric/electrostrictive element having one end fixed to said driven member with an inertial member fixed to the opposite end of the piezoelectric/electrostrictive element.

US4907158 discloses a method for manipulating cells in a cell culture whereby a television image of the cells is produced and a movable marker is superposed on the image. An operator can position the marker on a particular cell to be injected and the coordinates of the marked cell are used by a computer to advance a capillary point to the marked cell and thus manipulate it.

Once a heterologous nucleic acid had been introduced into a recipient cell, for example a fibroblast or a spermatozoon, the nuclei must be transferred to recipient ovum or an enucleated cytoplast thereof. Avian ova, however, because of the optically opaque yolk underlying the oocyte or germinal disk, present unique limitations to microinjection that are not encountered when microinjecting with other, less optically dense cells such as mammalian ova. The yolk restricts evaluation of the penetration of the larger embryonic tissue by micropipette by using an optical microscope and transmitted light. Also, where microinjection has been achieved, the incubation for the development of the embryo has been *ex ova,* requiring labor-intensive maintenance of artificial eggs until hatching. The success rate in terms of viable and healthy chicks by these procedures is as low as about 10-15%.

What is needed, therefore, is a method of microinjection into the avian germinal disk of an avian ovum that allows a micropipette to be placed accurately and rapidly within the germinal disk of an avian egg for the delivery thereto of a heterologous cell nucleus or nucleic acid, and to return the recombinant ovum to a hen for the formation and laying of a hard-shelled egg.

What is further needed is a microinjection apparatus for the delivery of a heterologous nucleic acid to the germinal disk overlaying the yolk of an avian egg.

What is also needed is an apparatus for the microinjection of the germinal disk of an avian ovum wherein the penetration of the germinal disk by a micropipette is monitored by a microscope.

### Summary of the Invention

Briefly described, the assembly of the present invention comprises an optical microscope, a microinjection system and an oblique macro-monitoring unit for the microinjection of an avian germinal disk. The assembly of the present invention allows the operator to monitor the extent of the microinjection into an avian germinal disk without interference from the optically opaque egg yolk.

In one aspect of the present invention, the microinjection system comprises a micromanipulator and a piezo-electric oscillator, each operably connected to a micropipette. The microscope may use transmitted light to monitor micropipette manipulation for filling the lumen thereof with a fluid, the fluid including a heterologous nucleic acid, such as a cell nucleus, a spermatozoon or an isolated nucleic acid. The microscope also has an incident light beam to place the ovum in a predetermined position. In this aspect of the present invention, the relative position of the micropipette and the avian germinal disk of the ovum are monitored by the oblique macro-monitoring unit comprising a macro lens, an electronic camera connected thereto and a monitoring unit.

Another aspect of the present invention is a method of delivering a heterologous nucleic acid to an avian germinal disk wherein the avian ovum is removed from a female bird and disposed in an incident light beam of a microscope.

Additional objects and aspects of the present invention will become more apparent upon review of the detailed description set forth below when taken in conjunction with the accompanying figures, which are briefly described as follows.

### Brief Description of the Figures

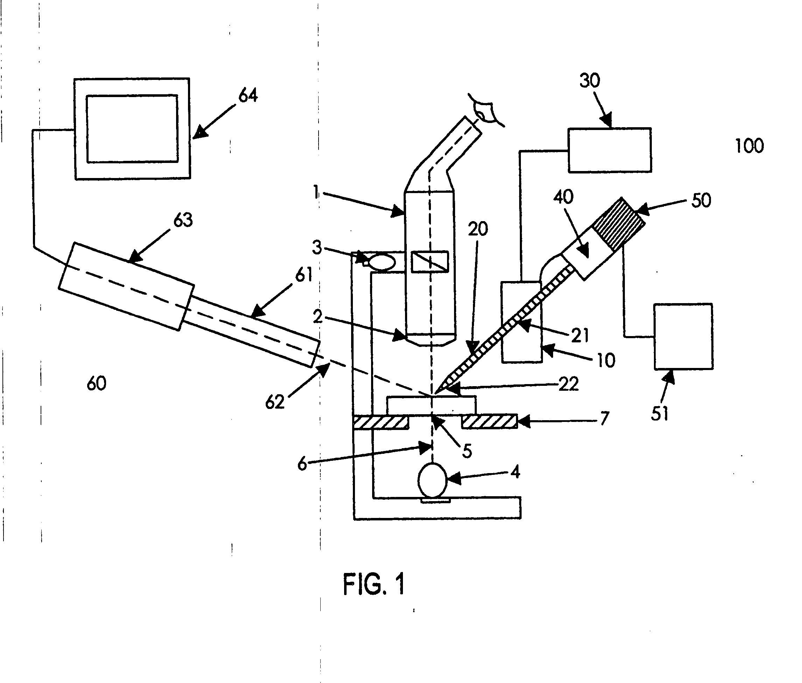

Fig. 1 illustrates a microinjection assembly according to the present invention for microinjecting the germinal disk of an avian ovum.

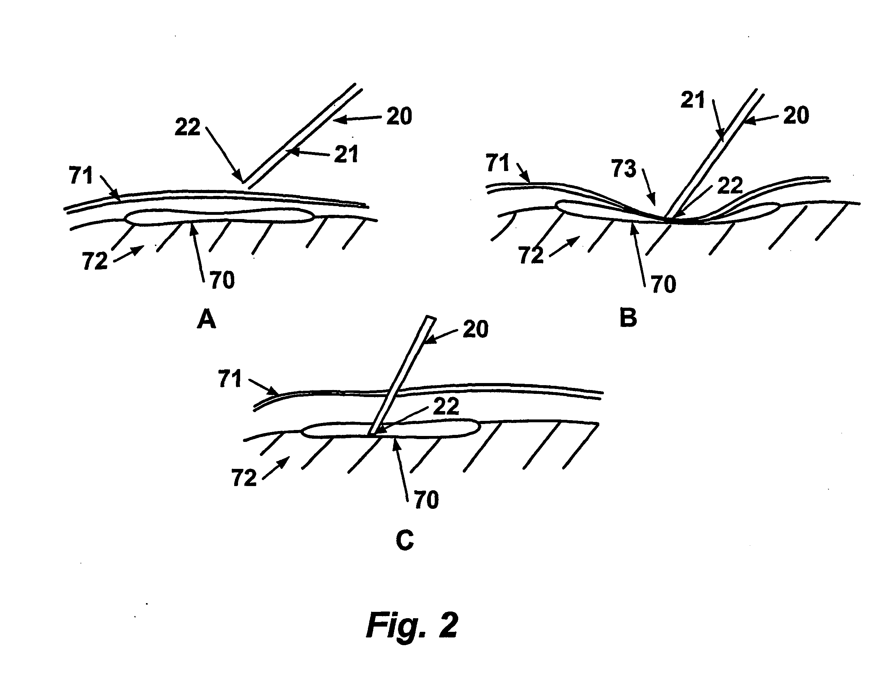

Figs. 2A - 2C illustrate the positioning of a micropipette made according to the present invention. Fig.2A shows the micropipette positioned over the vitelline membrane of an avian ovum and over the underlying germinal disk. Fig. 2B illustrates the indentation of the vitelline membrane of an avian ovum by depressing a micropipette. Fig. 2C illustrates the insertion of a micropipette into the germinal disk of an avian ovum after penetrating the overlying vitelline membrane.

### Detailed Description of the Invention

Reference will now be made in detail to the presently preferred embodiments of the invention, one or more examples of which are illustrated in the accompanying drawings. Each example is provided by way of explanation of the invention, not limitation of the invention. For instance, features illustrated or described as part of one embodiment can be used in another embodiment to yield yet another embodiment. It is intended that the present invention covers such modifications, combinations, additions, deletions and variations as fall within the scope of the appended claims and their equivalents.

Throughout this application various publications are referenced.

For convenience, certain terms employed in the specification, examples, and appended claims are collected here.

### Definitions

The term "animal" as used herein refers to all vertebrate animals, including birds. It also includes an individual animal in all stages of development, including embryonic and fetal stages.

The term "avian" as used herein refers to any species, subspecies or race of organism of the taxonomic class *aves,* such as, but not limited to, chicken, turkey, duck, goose, quail, pheasants, parrots, finches, hawks, crows and ratites including ostrich, emu and cassowary. The term includes the various know strains of *Gallus gallus,* or chickens, (for example, White Leghorn, Brown Leghorn, Barred-Rock, Sussex, New Hampshire, Rhode Island, Ausstralorp, Minorca, Amrox, California Gray, Italian Partidge-colored), as well as strains of turkeys, pheasants, quails, duck, ostriches and other poultry commonly bred in commercial quantities.

The term "germinal disk" as used herein refers to the active cytoplasmic area on the yolk of an unfertilized or fertilized (Stage I-IV) ovum before cleavage of the entire cytoplasmic area. The "germinal disk," therefore, may be a single nucleated cytoplasmic unit prior to fertilization or a multicellular blastodisc.

The term "blastoderm" as used herein refers to the avian embryonic stage wherein the *area pellucida* is complete (Stage X), the blastodermal cell layer being detached from the underlying yolk.

The term "Stage X embryo" as used herein refers to the blastodermal stage of the avian embryonic developmental cycle at the point where the hard-shell egg is laid. Ovulation in the chicken occurs 20-30 minutes after the laying of an egg. The ovum comprises a massive optically opaque yolk, on top of which is a 2-3mm diameter cytoplasmic or germinal disk.

The terms "ovum" and "oocyte" are used interchangeably herein. Although only one ovum matures at a time, an animal is born with a finite number of ova. In avian species, such as a chicken for example, ovulation, which is the shedding of an egg from the ovarian follicle, occurs when the brain's pituitary gland releases a luteinizing hormone, LH. Mature follicles form a stalk or pedicle of connective tissue and smooth muscle. Immediately after ovulation the follicle becomes a thin-walled sac, the post-ovulatory follicle. The mature ovum erupts from its sac and starts its journey through the oviduct. Eventually, the ovum enters the infundibulum where fertilization occurs. Fertilization must take place within 15 minutes of ovulation, before the ovum becomes covered by albumen. During fertilization, sperm (avians have polyspermic fertilization) penetrate the germinal disk, the small white spot on the top side of the yolk where the embryo will develop. When the sperm lodges within this germinal disk, an embryo begins to form. It is now known as a "blastoderm" or "zygote." The fertilized ovum descends the oviduct where the outer albumen and the shell membranes are deposited around the ovum. The hard shell is deposited once the ovum has reached the uterus. In the uterus, rotation of the egg governs the orientation of the embryo in the egg. See Eyal-Giladi, 1991, Revs. Poultry Biol. 3: 143-166, incorporated herein by reference in its entirety.

The zygote (germinal disk) begins to cleave once the ovum enters the uterus, with a series of 5-6 divisions over a two-hour period, whereupon the central cells detach from the underlying yolk. The space between the cells and the yolk is the sub-blastodermic cavity. After about 11 hours, the germinal disk is a 5-6 cell thick blastoderm (Stage V of development). In the succeeding Stages VII-X, the cells closest to the yolk slough and fall to the yolk surface (Stage VIII) to leave a one-cell thick layer in the center of the blastoderm, the *area pellucida* (Stage X), whereupon the egg is laid. At Stage X, the blastoderm has predestined anterior and posterior ends for the developing embryo.

The terms "gene" or "genes" as used herein refer to nucleic acid sequences (including both RNA or DNA) that encode genetic information for the synthesis of a whole RNA, a whole protein, or any portion of such whole RNA or whole protein. Genes that are not naturally part of a particular organism's genome are referred to as "foreign genes," "heterologous genes" or "exogenous genes" and genes that are naturally a part of a particular organism's genome are referred to as "endogenous genes." The term "gene product" refers to RNAs or proteins that are encoded by the gene. "Foreign gene products" are RNA or proteins encoded by foreign genes and "endogenous gene products" are RNA or proteins encoded by endogenous genes. "Heterologous gene products" are RNAs or proteins encoded by foreign, heterologous genes and that, therefore, are not naturally expressed in the cell.

The term "nucleic acid" as used herein refers to any natural or synthetic linear or sequential array of nucleotides and nucleosides, for example cDNA, genomic DNA, mRNA, tRNA, oligonucleotides, oligonucleosides and derivatives thereof. For ease of discussion, such nucleic acids may be collectively referred to herein as "constructs," "plasmids," or "vectors." Representative examples of bacterial plasmid vectors include expression, cloning, cosmid and transformation vectors such as, but not limited to, pBR322, animal viral vectors such as, but not limited to, modified adenovirus, influenza virus, adeno-associated virus, polio virus, pox virus, retrovirus, and the like, vectors derived from bacteriophage nucleic acid, and synthetic oligonucleotides such as chemically synthesized DNA or RNA. The term "nucleic acid" further includes modified or derivatised nucleotides and nucleosides such as, but not limited to, halogenated nucleotides such as, but not only, 5-bromouracil, and derivatised nucleotides such as biotin-labeled nucleotides.

The term "isolated nucleic acid" as used herein refers to a nucleic acid with a structure (a) not identical to that of any naturally occurring nucleic acid or (b) not identical to that of any fragment of a naturally occurring genomic nucleic acid spanning more than three separate genes, and includes DNA, RNA, or derivatives or variants thereof. The term covers, for example, (a) a DNA which has the sequence of part of a naturally occurring genomic molecule but is not flanked by at least one of the coding sequences that flank that part of the molecule in the genome of the species in which it naturally occurs; (b) a nucleic acid incorporated into a vector or into the genomic nucleic acid of a prokaryote or eukaryote in a manner such that the resulting molecule is not identical to any vector or naturally occurring genomic DNA; (c) a separate molecule such as a cDNA, a genomic fragment, a fragment produced by polymerase chain reaction (PCR), ligase chain reaction (LCR) or chemical synthesis, or a restriction fragment; (d) a recombinant nucleotide sequence that is part of a hybrid gene, i.e., a gene encoding a fusion protein, and (e) a recombinant nucleotide sequence that is part of a hybrid sequence that is not naturally occurring.

The term "fragment" as used herein to refer to a nucleic acid (e.g., cDNA) refers to an isolated portion of the subject nucleic acid constructed artificially (e.g., by chemical synthesis) or by cleaving a natural product into multiple pieces, using restriction endonucleases or mechanical shearing, or a portion of a nucleic acid synthesized by PCR, DNA polymerase or any other polymerizing technique well known in the art, or expressed in a host cell by recombinant nucleic acid technology well known to one of skill in the art. The term "fragment" as used herein may also refer to an isolated portion of a polypeptide, wherein the portion of the polypeptide is cleaved from a naturally occurring polypeptide by proteolytic cleavage by at least one protease, or is a portion of the naturally occurring polypeptide synthesized by chemical methods well known to one of skill in the art.

The terms "nucleic acid vector" or "vector" as used herein refer to a natural or synthetic single or double stranded plasmid or viral nucleic acid molecule that can be transfected or transformed into cells and replicate independently of, or within, the host cell genome.

The term "plasmid" as used herein refers to a small, circular DNA vector capable of independent replication within a bacterial or yeast host cell.

The term "cytoplast" as used herein refers to a chromosome-free recipient cell, wherein chromosomal removal is referred to as enucleation when the nucleus or chromosomes organized in a Metaphase plate of a cell are removed or destroyed.

The term "recombinant cell" refers to a cell that has a new combination of nucleic acid segments that are not covalently linked to each other in nature. A new combination of nucleic acid segments can be introduced into an organism using a wide array of nucleic acid manipulation techniques available to those skilled in the art. The recombinant cell can harbor a vector that is extragenomic. An extragenomic nucleic acid vector does not insert into the cell's genome. A recombinant cell can further harbor a vector or a portion thereof that is intragenomic. The term "intragenomic" defines a nucleic acid construct incorporated within the recombinant cell's genome.

The term "recombinant nucleic acid" as used herein refers to combinations of at least two nucleic acid sequences that are not naturally found in a eukaryotic or prokaryotic cell. The nucleic acid sequences may include, but are not limited to nucleic acid vectors, gene expression regulatory elements, origins of replication, sequences that when expressed confer antibiotic resistance, and protein-encoding sequences. The term recombinant polypeptide" is meant to include a polypeptide produced by recombinant DNA techniques such that it is distinct from a naturally occurring polypeptide either in its location, purity or structure. Generally, such a recombinant polypeptide will be present in a cell in an amount different from that normally observed in nature.

The term "male germ cells" as used herein refers to spermatozoa (i.e., male gametes) and developmental precursors thereof. In the sexually mature male vertebrate animal, there are several types of cells that are precursors of spermatozoa, and which can be genetically modified, including the primitive spermatogonial stem cells, known as A0/As, which differentiate into type B spermatogonia. The latter further differentiate to form primary spermatocytes, and enter a prolonged meiotic prophase during which homologous chromosomes pair and recombine. Useful precursor cells at several morphological/developmental stages are also distinguishable: preleptotene spermatocytes, leptotene spermatocytes, zygotene spermatocytes, pachytene spermatocytes, secondary, spermatocytes, and the haploid spermatids. The latter undergo further morphological changes during spermatogenesis, including the reshaping of their nucleus, the formation of aerosome, and assembly of the tail. The final changes in the spermatozoa (i.e., male gamete) take place in the genital tract of the female, prior to fertilization.

The term "transgenic animal" as used herein refers to any avian species, including, but not limited to, the chicken, in which one or more of the cells of the bird contain heterologous nucleic acid introduced by way of human intervention, such as by transgenic techniques well known in the art. The nucleic acid is introduced into a cell, directly or indirectly by introduction into a precursor of the cell, by way of deliberate genetic manipulation, such as by sperm-mediated or restriction-enzyme mediated integration, microinjection or by infection with a recombinant virus. The term "genetic manipulation" does not include classical cross-breeding, or *in vitro* fertilization, but rather is directed to the introduction of a recombinant DNA molecule. This molecule may be integrated within a chromosome, or it may be extrachromosomally replicating DNA. In the typical transgenic animal, the transgene causes cells to express a recombinant form of an immunoglobulin polypeptide or a variant polypeptide thereof.

As used herein, the term "transgene" means a nucleic acid sequence that is partly or entirely heterologous, i.e., foreign, to the transgenic animal or cell into which it is introduced, or, is homologous to an endogenous gene of the transgenic animal or cell into which it is introduced, but which is designed to be inserted, or is inserted, into the animal's genome in such a way as to alter the genome of the cell into which it is inserted (e.g., it is inserted at a location which differs from that of the natural gene or its insertion results in a knockout). A transgene can include one or more transcriptional regulatory sequences and any other nucleic acid, such as introns, that may be necessary for optimal expression of a selected nucleic acid.

The term "donor cell" as used herein refers to the source of the nuclear structure that is transplanted to the recipient enucleated cytoplast. All cells of normal karyotype, including embryonic, fetal, and adult somatic cells may be nuclear donors. The use of non-quiescent cells as nuclear donors has been described by Cibelli et al., (1998, Science 280: 1256-8).

The term "recipient cell" as used herein refers to the enucleated recipient cell, preferably an enucleated metaphase I or II oocyte an enucleated preactivated oocyte or a pronuclear stage egg. Enucleation may be accomplished by splitting the cell into halves, aspirating the metaphase plate, pronucleus or pronuclei, or even by irradiation. Enucleation may be done through two-photon laser-mediated ablation. TPLSM could be used to guide mechanical enucleation.

The term "TPLSM" as used herein refers to two-photon laser scanning microscopy. TPLSM is based on two-photon excited fluorescence in which two photons collide simultaneously with a fluorescent molecule. Their combined energy is absorbed by the fluorophore, inducing fluorescent emission, detected by a photomultiplier tube and converted into a digital image. (See Squirrell et al., 1999, Nature Biotechnol. 17: 763-7 and Piston et al., 1999, Trends Cell Biol. 9: 66-9). TPLSM allows for the generation of images of living, optically dense structures for prolonged periods of time, while not affecting their viability. TPLSM utilizes biologically innocuous pulsed near-infrared light, usually at a wavelength of about 700 nm to about 1000 nm, which is able to penetrate deep into light-scattering specimens. TPLSM may employ different lasers, such as a mode-locked laser, where the wavelength is fixed, or a tunable laser that can be tuned to wavelengths between about 700 nm and about 1000 nm, depending upon the range of emission of the dye used. For DAPI and Hoescht 33342 dyes, 750-830 nm is suitable. New fluorophores are being produced with different ranges of emission and the invention is not limited to the presently available dyes and their respective emission ranges. Furthermore, lasers used in TPLSM can be grouped into femtosecond and picosecond lasers. These lasers are distinguished by their pulse duration. A femtosecond laser is preferred since it is particularly suitable for visualization without harming the specimen.

### Abbreviations

Abbreviations used in the present specification include the following: cDNA, DNA complementary to RNA; mRNA, messenger RNA; tRNA, transfer RNA; nt, nucleotide(s); TPLSM, two photon laser scanning microscopy; REMI, restriction enzyme mediated integration.

The present invention is directed to providing an assembly for the delivery of an isolated cell nucleus, a spermatozoon or a fluid having , a nucleic acid therein, by microinjection into an avian germinal disk. The present invention is further directed to providing methods of microinjecting a fluid having a nucleic acid therein, into an avian germinal disk. More specifically, the present invention provides methods for delivering a heterologous nucleic acid to an avian germinal disk, implanting the microinjected ovum into a hen wherein the hard-shell egg is then formed, laid, and the embryo develops and hatches as a chick.

With reference, therefore, to Fig. 1, the microinjection assembly of the present invention includes a microscope **1,** a microinjection system **100** and an obliquely angled macro monitoring unit **60,** wherein the microinjection system **100** is oriented with respect to the microscope **1** so as to be able to microinject an object **5** disposed on the microscope **1,** and wherein the macro monitoring unit **60** is oriented to monitor the microinjection of the object **5.**

The microscope **1** is operably connected to an objective **2.** The microscope **1** also has an optical axis **6** passing through the objective **2,** that may be coaxial with an incident light source **3,** generally an incident light beam, and a stage **7.** The optical microscope **1** of the microinjection assembly of the present invention may be any optical microscope wherein the objective **2** can be positioned over the object **5** to be viewed. The microscope objective **2** has a magnification of between about x5 to about x50, selected according to the size of the object being viewed. For example, the highest (about x50) magnification may be used to observe the loading of a micropipette with a cell nucleus or a suspension of spermatozoa. The lowest (about x5) magnification, for example, may be used for observing the microinjection of an avian ovum. Optionally, the microscope **1** may further comprise a transmitted light source **4,** wherein the light from the transmitted light source **4** is directed through an object 5 disposed on the stage **7** of the microscope **1.**

It is contemplated to be within the scope of the present invention for the object **5** to be an avian ovum removed from a female bird after ovulation and before deposition of albumen and shell thereon, or a vessel containing a fluid having an isolated nucleic acid or cell nucleus that is to be injected into an avian ovum (or germinal disk thereof).

The microinjection system **100** of the microinjection assembly according to the present invention comprises a micromanipulator **10** operably connected to a micropipette **20** wherein the micropipette **20** has a lumen **21** therein and a distal tip **22,** and optionally, is operably connected to a programmable control unit **30.** Preferably, the micromanipulator **10** can allow the micropipette **20** to be oriented to any position relative to the object **5** disposed on the stage **7** of the microscope **1.** Any micromanipulator **10** known to one **of** skill in the art may be incorporated into the microinjection system **100** of the present invention. The micromanipulator **10** may further comprise a pressure regulating system **40** such as a pump, for example, an air pump, a liquid pump, or a syringe pump that will allow the operator of the microinjection system **100** of the present invention to apply a positive or negative hydraulic pressure to the lumen **21** of the micropipette **20** so that a fluid may be drawn into, or ejected from, the lumen **21.**

The programmable control unit **30** operably connected to the micromanipulator **10** may store electronic signals that defme a selected position and angle of the micropipette **20** relative to a predetermined point, such as a predetermined point situated on or near an object **5** disposed on the stage **7** of the microscope **1.** The micropipette **20** may then be moved from the predetermined point, and returned to the same, by operating the programmable control unit **30.**

The microinjection system **100** of the present invention also further comprises a piezo-electric oscillator **50** operably connected to the micropipette **20** and to a control unit **51**. An example of a suitable oscillator unit that may be used in the microinjection assembly of the present invention is the PIEZODRILL^{™} Inertial Impact Drill (Burleigh Instruments, Inc.). Operation of the piezo-electric oscillator **50** will impart vibrations of preselected frequency, amplitude and bandwidth to the distal tip **22** of the micropipette **20** directed longitudinally to the lumen **21** of the micropipette **20,** or in a direction normal to the lumen **21.** The speed of the drilling is controlled by the frequency of oscillations imparted to the distal tip **5** of the micropipette **20.** The frequencies contemplated by the present invention range from about 1 Hz to about 100 Hz, preferably between about 1 Hz and about 25 Hz. The strength of the oscillations is controlled by the amplitude of the vibrations and may be in the range of about 1 volt to about 100 volts. Bandwidth of the oscillations regulate the sharpness of the vibrational pulse imparted to the micropipette **20.**

The microinjection assembly of the present invention further comprises an obliquely angled macro monitoring unit **60** comprising a macro lens **61** having an optical axis **62** directed to the object **5** disposed on the stage **7** of the microscope **1,** and at an oblique angle to the surface of the object **5.** The macro lens **61** is operably connected to an electronic camera **63,** and thereby to a monitor **64** that displays the image generated by the macro lens **61** and the electronic camera **63.** The macro lens **61** may be focused by adjusting the internal lens configuration thereof, or by moving the macro lens **61** in a direction along the optical axis 62, to or from the object **5.**

Any micropipette **20** suitable for the microinjection of an avian ovum may be used in the microinjection assembly of the present invention. The internal diameter of the micropipette **20** may be selected as a function of the size of a cell nucleus to be transferred to an avian embryonic cell. For example, the preferred internal diameter of the micropipette is between about 10 µm and about 15 µm when a nucleus to be transferred to an enucleated avian ovum has been isolated from a blastodermal cell. The internal diameter may be between about 4 µm and about 8 µm, however, when the nucleus has been obtained from a fibroblast, or is a spermatozoon.

The microinjection assembly of the present invention is useful for delivering a fluid containing an isolated cell nucleus, a spermatozoon or an isolated nucleic acid such as, but not limited to, a plasmid or a viral vector, to the cytoplasm or cytoplast of an avian embryonic cell, an avian ovum (oocyte) or an avian embryo. First, an avian ovum, preferably having a pre-stage X germinal disk, is surgically removed from an ovulating hen between about 30 minutes and about 2 hours of the previous laying of a hard-shell egg. This surgically removed avian ovum can then be placed in a specimen container, such as a glass dish, and placed on the stage **7** of the optical microscope **1.**

The lumen **21** of the micropipette **20** is loaded with a fluid that is to be injected into the avian ovum, avian embryonic cell or cytoplast. Using the transmitted light source **4** of the microscope to illuminate the micropipette **20,** the distal tip **22** of the micropipette **20** can be positioned to remove a nucleus from a donor cell, to gather spermatozoa or to be loaded with a fluid containing an isolated nucleic acid such as, for example, a plasmid or viral vector. The transmitted light source **4** allows the assembly operator to monitor the extent of the micropipette charging or to manipulate cells to remove the nucleus therefrom. The micropipette **20** may also further be charged with an inert liquid, such as FLOURINERT^{™} that will transmit piezo-electric induced oscillations from the piezo-electric oscillator **50** to the distal tip **22** of the micropipette **20.** All fluids and manipulated cell nuclei may be drawn into the micropipette by a pump **40** operably connected to the micropipette **20,** wherein the pump **40** is capable of positively or negatively regulating the hydraulic pressure in the lumen **21** of the micropipette **20** to ingress or eject the fluid respectively.

Referring to Figs. 2A-2C, wherein the piezo-electric oscillation-induced drilling of a vitelline membrane is shown, once the micropipette **20** is loaded, the surgically excised egg is placed on the stage **7** of the microscope **1** and illuminated with an incident beam of light. In one embodiment of the microinjection system of the present invention, the incident beam of light is coaxial with the optical axis of the microscope objective. In another embodiment of the microinjection system of the present invention, the incident beam of light is angled from the optical axis **6** of the objective **2.** Placement of the germinal disk **70** to a predetermined position relative to the microscope **1,** and thereby in the optical axis **62** of the macro monitoring unit **60,** is facilitated by first positioning the germinal disk **70** in the incident light beam of the microscope **1.**

Referring now to Fig. 2A, when the germinal disk **70** of the avian egg is positioned in, and illuminated by, the incident light beam, the micropipette **20** is moved to a preprogrammed selected position whereby the distal tip **22** of the micropipette **20** is over the area of the germinal disk **70** and therefore optimally placed for the insertion of the micropipette **20** into the germinal disk **70.** The distal tip **22** of the micropipette **20** is then pressed onto the vitelline membrane **71** of the avian egg, to a depth of about 20 µm below the general plane of the membrane, as shown in Fig. 2B. The vitelline membrane **71** resists penetration by the micropipette **20** and therefore the distal tip **22** indents the vitelline membrane **71** without piercing the membrane **71.**

The depth of the indentation **73** formed by the pressure of the distal tip **22** of the micropipette 20 on the vitelline membrane **71** can be determined by at least two methods. The micropipette may be pre-marked about 20 µm from the distal tip **22.** When the mark is about level with the general plane of the membrane, the distal tip **22** will enter the germinal disk **70** once the vitelline membrane **71** is penetrated. The distance for the micropipette **20** to be depressed may also be controlled by measuring the micropipette **20** movement against a precalibrated scale on the monitor **64** of the oblique macro-monitoring unit **60.**

The movement of the micropipette **20** relative to an avian germinal disk **70** is monitored by the obliquely angled macro monitoring unit **60,** comprising a focusable macro lens **61** capable of delivering a focused magnified image of the avian germinal disk **70** to an electronic camera **63** for display by a monitor **64.** The oblique angle of the macro lens **61** shows the depth of movement of the micropipette **20** relative to the vitelline membrane **71** and the degree of indentation thereof, more distinctly than if a vertical microscope objective **2** is used to monitor the microinjection.

Pulses of piezo-electric induced oscillations are applied to the micropipette **20** once it is in contact with the indented vitelline membrane **71.** The vibrating distal tip **22** of the micropipette **20** drills through the vitelline membrane **71,** Successful penetration, and therefore placement of the distal tip **22** at a desired position within the avian germinal disk **70,** is signaled by the vitelline membrane, **71** moving suddenly to its non-indented conformation, as shown in Fig. 2C. The fluid contents of the micropipette **20** can then be injected into the germinal disk **70** by positive hydraulic pressure exerted on the lumen **21** and the contents therein, by the pressure-regulating system **40,**

Described herein are methods for producing a transgenic bird, such as, but not limited to, a chicken, by introducing a transgene to an avian germinal disk using a viral or a non-viral vector, by sperm-mediated gene transfer, integration or the by nuclear transfer via two-photon visualization and optionally, laser-mediated ablation, and ovum transfer and the like. Transgenic avians produced by the methods may have the ability to lay eggs that contain one or more desired heterologous protein(s) such as, for example, an immunoglobulin light or heavy chain, an antibody, or variant thereof.

Transgenes may be introduced into the ovum of a bird, according to the present invention, by nuclear transfer via two-photon visualization and ablation, wherein the nuclear donor contains a desired heterologous DNA sequence in its genome. One of ordinary skill in the art will be able to readily adapt conventional methods to insert the desired transgene into the genome of the nuclear donor prior to injection of the nuclear donor into a recipient cytoplast. For example, a vector that contains one or more transgene(s), encoding at least one polypeptide chain of an antibody, may be delivered into the nuclear donor cell through the use of a delivery vehicle. The transgene is then transferred along with the nuclear donor into the recipient ovum. Following zygote reconstruction by the methods of the present invention, the ovum is transferred into the reproductive tract of a recipient hen. In a preferred embodiment of the present invention, the ovum is transferred into the infundibulum of the recipient hen. After reconstruction, the embryo containing the transgene develops inside the recipient hen and travels through the oviduct of the hen where it is encapsulated by natural egg white proteins and a natural egg shell. The egg is laid and can be incubated and hatched to produce a transgenic chick. The resulting transgenic chick will carry one or more desired transgene(s) in its germ line. Following maturation, the transgenic avian may lay eggs that contain one or more desired heterologous protein(s) that can be easily harvested.

Methods for transfection of somatic cell nuclei are well known in the art and include, by way of example, the use of retroviral vectors, retrotransposons, adenoviruses, adeno-associated viruses, naked DNA, lipid-mediated transfection, electroporation and direct injection into the nucleus. Such techniques, particularly as applied to avians, are disclosed by Bosselman (U.S, Patent No. 5,162,215), Etches (PCT Publication No. WO 99/10505), Hodgson (U.S. Patent No. 6,027,722), Hughes (U.S. Patent No. 4,997,763), Ivarie (PCT Publication No. WO 99/19472), MacArthur (PCT Publication No. WO 97/47739), Perry (U.S. Patent No. 5,011,780), Petitte (U.S. Patent Nos. 5,340,740 and 5,656,749), and Simkiss (PCT Publication No. WO 90/11355), the disclosures of which are incorporated by reference herein in their entireties.

Also described is a cloned bird using nuclear transfer methods employing two-photon visualization. The steps in nuclear transfer include, but are not limited to, the preparation of a cytoplast, donor cell nucleus (nuclear donor) isolation and transfer to the cytoplast to produce a reconstructed embryo, optional culturing of the reconstructed embryo, and embryo transfer to a synchronized host animal.

In preferred embodiments of the invention, the animal is an avian including, but not limited to, chickens, ducks, turkeys, quails, pheasants and ratites. In this method, a fertilized or unfertilized egg is removed from a bird and manipulated *in vitro*, wherein the genetic material of the egg is visualized and removed and the ablated nucleus replaced with a donor nucleus. Optionally, the donor nucleus may be genetically modified with, for example, a transgene encoding an exogenous polypeptide. Two-photon laser scanning microscopy (TPLSM) can be used to visualize the nuclear structures. Following visualization, the nucleus in the recipient cell, such as a fertilized or unfertilized egg, is removed or ablated, optionally using TPLSM.

TPLSM produces non-invasive, three-dimensional, real-time images of the optically dense avian egg. Visualization of the metaphase plate or pronucleus in avian eggs during nuclear transfer has been prevented by the yolk. Two-photon imaging with femtosecond lasers operating in the near infrared, however, allows visualization of nuclear structures without damaging cellular constituents. Prior to visualization, specimens may be incubated or injected with DNA-specific dyes such as DAPI (4', 6'-diamidino-2-phenylindole hydrochloride) or Hoescht 33342 (bis-benzimide), the albumen capsule is removed and the ovum placed in a dish with the germinal disk facing the top. Remnants of the albumen capsule are removed from the top of the germinal disk.

An aqueous solution, for example phosphate-buffered saline (PBS), is added to prevent drying of the ovum. A cloning cylinder is placed around the germinal disk and DAPI in PBS is added to the cylinder. Alternatively, a DAPI-PBS solution may be injected into the germinal disk with a glass pipette, whereupon the dye enters the nuclear structures. For dye injection, removal of the albumen capsule is not necessary, whereas injection of nuclei into the disk is facilitated in the absence of the capsule.

Images of the inside of the early avian embryo can be generated through the use of TPLSM. Visualization may be performed after about 10 to 15 minutes of incubation or about 10 minutes after dye injection. During visualization, the germinal disk is placed under the microscope objective and the pronuclear structures are searched within the central area of the disk using relatively low laser powers of about 3-6 milliwatts. Once the structures are found they may be ablated by using higher laser power or be mechanically removed, guided by TPLSM.

Nuclear transfer also requires the destruction or enucleation of the pronucleus before a nuclear donor can be introduced into the oocyte cytoplast. Two-photon laser-mediated ablation of nuclear structures provides an alternative to microsurgery to visualize the pronucleus lying about 25µm beneath the ovum's vitelline membrane within the germinal disk. Higher laser powers than those used for imaging are used for enucleation, with minimal collateral damage to the cell. The wavelength for ablation generally ranges from about 700 nm to about 1000 nm, at about 30 to about 70 milliwatts. TPLSM and two-photon laser-mediated ablation are more efficient than alternative methods because they are less operator dependent and less invasive, which results in improved viability of the recipient cell.

A cultured somatic cell nucleus (nuclear donor) may then be injected into the enucleated recipient cytoplast by the microinjection assembly of the present invention. The donor nucleus is introduced into the germinal disk through guided injection using episcopic illumination (i.e., light coming through the objective onto the sample). The reconstructed zygote may then be surgically transferred to the oviduct of a recipient hen to produce a hard-shell egg. Alternatively, the reconstructed embryo may be cultured for 24 hours and screened for development prior to surgical transfer.

The egg can be harvested after laying and before hatching of a chick, or further incubated to generate a cloned chick, optionally genetically modified. The cloned chick may carry a transgene in all or most of its cells. After maturation, the transgenic avian may lay eggs that contain one or more desired heterologous protein(s). The cloned chick may also be a knock-in chick expressing an alternative phenotype or capable of laying eggs having a heterologous protein therein, The reconstructed egg may also be cultured to term using the *ex ovo* method described by Perry *et al.* (*supra*) and incorporated herein by reference in its entirety.

The replacement of the recipient cell's nucleus with the donor cell's nucleus results in a reconstructed zygote. Preferably, the cytoplasmic membrane of the cell used as nuclear donor is disrupted to expose its nucleus to the ooplasm of the recipient cytoplast. The nuclear donor may be injected into the germinal disk, where it undergoes reprogramming and becomes the nucleus of the reconstructed one-cell embryo.

Also described is a method of producing a cloned bird comprising nuclear transfer in combination with ovum transfer. Two-photon visualization and ablation may be used to perform nuclear transfer, as described above. Accordingly, the replacement of the recipient cell's nucleus with the donor cell's nucleus results in a reconstructed zygote. Preferably, pronuclear stage eggs are used as recipient cytoplasts already activated by fertilization. Alternatively, unactivated metaphase II eggs may serve as recipient cytoplast and activation induced after renucleation. The ovum may then be cultured via ovum transfer, wherein the ovum containing the reconstructed zygote is transferred to a recipient hen. The ovum is surgically transferred into the oviduct of the recipient hen shortly after oviposition. This is accomplished according to normal husbandry procedures (oviposition, incubation, and hatching; see Tanaka *et al., supra*).

Alternatively, the ovum may be cultured to Stage X prior to transfer into a recipient hen. More specifically, reconstructed Stage I embryos are cultured for 24-48 hours to Stage X. This allows for developmental screening of the reconstructed embryo prior to surgical transfer. Stage I embryos are enclosed within a thick albumen capsule. In this novel procedure, the albumen capsule is removed, after which the nuclear donor is injected into the germinal disk using the microinjection assembly and the methods of use thereof, of the present invention. Subsequently, the capsule and germinal disk are recombined by placing the thick capsule in contact with the germinal disk on top of the yolk. Embryos develop to Stage X at similar rates as those cultured with their capsules intact. At Stage X of development, the embryo is transferred to the oviduct of a recipient hen.

Once transferred, the embryo develops inside the recipient hen and travels through the oviduct of the hen where it is encapsulated by natural egg white proteins and a natural egg shell. The egg that contains endogenous yolk and an embryo from another hen, is laid and can then be incubated and hatched like a normal chick. The resulting chick may carry a transgene in all or most of its cells. Preferably, the transgene is at least in the oviduct cells of the recipient chick. Following maturation, the cloned avian may express a desired phenotype or may be able to lay eggs that contain one or more desired heterologous protein(s).

Although preferred embodiments of the invention have been described using specific terms, devices, and methods, such description is for illustrative purposes only. The words used are words of description rather than of limitation. The present invention is further illustrated by the following examples, which are provided by way of illustration and should not be construed as limiting.

### Example 1: Preparation of the Recipient Cytoplast by TPLSM

*Incubation:* Ova were isolated from euthanized hens between 2-4 hours after oviposition of the previous egg. Alternatively, eggs were isolated from hens whose oviducts have been fistulated (Gilbert and Woodgush, 1963, J. of Reprod. and Fertility 5: 451-453) and (Pander et al., 1989, Br. Poult. Sci. 30: 953-7).
   Before generating images of the avian early embryo, DNA was incubated with a specific dye according to the following protocol. The albumen capsule was removed and the ovum placed in a dish with the germinal disk facing the top. Remnants of the albumen capsule were removed from the top of the germinal disk. Phosphate buffered saline (PBS) was added to the dish to prevent drying of the ovum. A cloning cylinder was placed around the germinal disk and 1.0µg/ml of DAPI in PBS was added to the cylinder. Visualization was performed after approximately 15 minutes of incubation.
*Injection:* Preparation of the egg was done as described for incubation. Following removal of the capsule, 10-50 nanoliters of a 0.1 µg/ml solution of DAPI in PBS was injected into the germinal disk using a glass pipette. Visualization was performed approximately 15 minutes after injection.
*Visualization:* Following incubation, images of the inside of the avian early embryo were generated through the use of TPLSM. The germinal disk was placed under the microscope objective, and the pronuclear structures were searched within the central area of the disk, to a depth of 60µm using low laser power of 3-6 milliwatts at a wavelength of 750 nm. Once the structures were found they were subsequently ablated.

### Example 2: Nuclear Ablation and Enucleation

Pronuclear structures were subjected to laser-mediated ablation. In these experiments, an Olympus 20x/0.5NA (Numerical Aperture) water immersion lens was used. The x and y planes to be ablated were defined with the two photon software, while the z plane (depth) was just under 10µm for this type of objective. Since the pronuclear structure was about 20 µm in diameter, the ablation comprised two steps (2 times 10µm). The focal point was lowered to visualize the remaining of the pronucleus, which was subsequently ablated. The laser power used to ablate the pronuclei was between about 30 to about 70 milliwatts at a wavelength of 750 nm. For the ablation experiments described above, the image was zoomed by a factor of 4 to 5, giving an area compression of 16-25 fold. Then the power was increased 10-12 fold for a total intensity increase of 160-300 fold compared to the visualization intensity of 3-6 milliwatts. The ablation intensity (power density) is the functional parameter, i.e. the power increase of 10-12 fold results in ablation power of 30-70 milliwatts, but the zoom factor compressed this power into an area 16-25x smaller giving a power density increase of 160-300 fold.

### Example 3: Nuclear transfer requires removal of the nucleus of the recipient ovum

Fertile White Leghorn ova were collected 1.5 hours after laying of an egg. The donor birds were sacrificed by cervical dislocation and the ova collected under aseptic conditions from the infundibulum or the anterior end of magnum.

Nuclei from blastodermal cells obtained from a stage X egg of a Barred Rock hen were microinjected into the center of the recipient germinal disks of White Leghorn ova without removal of the nuclei from the recipient cells. The ova were then transferred to a White Leghorn recipient hens for further development.

Feather color was used to determine positive acceptance of the donor nucleus by a nucleated recipient cell. Thus, White Leghorn birds have white feathers and Barred Rock have black feathers. An indication of a donor nucleus surviving in a nucleated cell would be offspring having black feathers, or black and white feathers (illustrating chimera formation).

Four all yellow chicks hatched, indicating that there must be damage to the first nucleus, of the recipient ovum, to have development with the donated nucleus. Although the donor nucleus may not be retained in an active form, this experiment shows that the microinjection of the second nucleus into a recipient ovum having a first nucleus still present does not preclude final and complete development off the ovum to a hatched chick.

### Example 4: Gamma Ray Irradiation for Nuclear Ablation

Fertile White Leghorn ovum donors are collected 2 - 2.5 hours after laying of an egg. The donor birds are sacrificed by cervical dislocation and the ova collected under aseptic conditions from the infundibulum or the anterior end of magnum.

Ova from the White Leghorn door birds are irradiated initially with 600 rads of gamma radiation. A nucleus from a blastodermal cell derived from a stage X egg of a Barred Rock hen is then microinjected into the center of the germinal disk. The microinjected ova are then transferred to White Leghorn recipient hens for further development.

Feather color is used to determine positive acceptance of the donor nucleus by a nucleated recipient cell. Thus, White Leghorn birds will have white feathers and Barred Rock will have black feathers. An indication of a donor nucleus surviving in a nucleated cell is offspring having black feathers, or black and white feathers (illustrating chimera formation).

The results indicate that damage to the nucleus of the recipient ovum by 600 rads of applied gamma radiation disturbs, but not halt, development of the embryo or prevent hatching thereof.

### Example 5: Preparation of the Nuclear Donor Cell and Isolation of the Donor Nucleus

Fibroblast cells in culture were trypsinized (0.25% Trypsin and 1µM EDTA), centrifuged twice in PBS containing 5% of fetal calf serum (PCS) and placed in a 60 mm plastic dish in PBS containing 5% of FCS. Using the microscope/micromanipulation unit described below, under transmission light, the nuclear donors were then isolated by repeated pipetting of the cells, which disrupted the cytoplasmic membrane and released the nucleus from inside the cell.

### Example 6: Preparation of the Reconstructed Zygote

A micromanipulation unit, comprising an IM-16 microinjector and a MM-188NE micromanipulator, both from Nikon/Marishige, were adapted to an upright Nikon Eclipse E800. This microscope was adapted to operate under both transmission and reflective light conditions. This unique configuration has allowed a morphological examination and preparation (i.e., isolation of the nuclei, as described above) somatic cells in suspension and to load the injection pipette using dry or water immersion lenses under diascopic illumination or transmitted light. This was followed by the prompt localization and positioning of the germinal disk under the microscope and subsequent guided injection of the somatic cells, using dry and long distance lenses under fiber optic as well as episcopic illumination (light coming from the side and through the objectives onto the sample respectively).

### Example 7: Ovum Transfer

At the time of laying, recipient hens are anesthetized by wing vein injection with pentobarbital (0.7 ml of a 68 mg/ml solution). At this time, the infundibulum is receptive to receiving a donor ovum but has not yet ovulated. Pentobarbital is the anesthetic of choice. Feathers are removed from the abdominal area, and the area is scrubbed with betadine, and rinsed with 70% ethanol. The bird is placed in a supine position and a surgical drape is placed over the bird with the surgical area exposed. An incision is made beginning at the junction of the sternal rib to the breastbone and running parallel to the breastbone. The length of the incision is approximately two inches. After cutting through the smooth muscle layers and the peritoneum, the infundibulum is located. The infundibulum is externalized and opened using gloved hands and the donor ovum is gently applied to the open infundibulum. The ovum is allowed to move into the infundibulum and into the anterior magnum by gravity feed. The internalized ovum is placed into the body cavity and the incision closed using interlocking stitches both for the smooth muscle layer and the skin. The recipient hen is returned to her cage and allowed to recover with free access to both feed and water. Recovery time for the bird to be up, moving and feeding is usually within 45 minutes of the operation's end. Eggs laid by the recipient hens are collected the next day, set, and incubated. They will hatch 21 days later.

Alternatively, a hen whose oviduct is fistulated allows the collection of eggs for enucleation (Gilbert and Woodgush, 1963, J. of Reprod. and Fertility 5: 451-453; Pancer et al., 1989, Br. Poult. Sci. 30: 953-7989). The transfer of the reconstructed embryo to a recipient hen for the production of a hard-shell egg is described by Wentworth, 1960, Poultry Science 39: 782-784. The first technique will be used to obtain ova for recipient cytoplasts and the latter to produce recipient hens to be used repeatedly for the transfer of reconstructed embryos.

## Claims

1. A microinjection assembly for the delivery of exogenous nucleic acid to an avian germinal disk, comprising:
an optical microscope;
a microinjection system; and
an oblique macro-monitoring unit.

2. A microinjection assembly as claimed in claim 1, wherein the microinjection system comprises a micropipette operably connected to a micromanipulator and an oscillator.

3. A microinjection assembly as claimed in claim 2, wherein the oscillator is a piezo-electric oscillator.

4. A microinjection assembly as claimed in any one of claims 1 to 3, wherein the optical microscope has an incident illumination system.

5. A microinjection assembly as claimed in claim 4, wherein the optical microscope has an objective with an optical axis, and wherein the incident illumination system is directed along the optical axis of the objective.

6. A microinjection assembly as claimed in any one of claims 2 to 5, wherein the micromanipulator is programmable.

7. A microinjection assembly as claimed in any one of claims 1 to 6, wherein the oblique monitoring unit comprises a lens operably connected to an electronic camera and a monitor unit.

8. A microinjection assembly as claimed in any one of claims 1 to 7, wherein the optical microscope has a transmitted light illumination system.

9. A microinjection assembly as claimed in any one of claims 1 to 8, wherein the microinjection system and the oblique monitoring unit are attached to the optical microscope.

10. A method for delivering exogenous nucleic acid to an avian germinal disk, comprising:
(a) providing a microinjection assembly comprising a an optical microscope, micropipette, an oblique macro-monitoring system and an avian germinal disk,
(b) loading the micropipette with a fluid having an exogenous nucleic acid therein;
(c) visualising the micropipette by monitoring the position of the micropipette relative to the avian germinal disc by the oblique macro-monitoring system; and
(d) delivering the fluid having the exogenous nucleic acid to the germinal disk.

11. A method as claimed in claim 10, wherein the germinal disk is present in an avian ovum or avian embryo.

12. A method as claimed in claim 11, further comprising delivering the germinal disk in the form of an avian embryo to a recipient avian female; allowing the avian embryo to be laid in a hard-shell egg; and allowing the embryo to develop and hatch as a chick.

13. A method as claimed in any one of claims 10 to 12, wherein the exogenous nucleic acid is an isolated nucleic acid selected from a plasmid, a viral vector and a linear nucleic acid, and wherein the exogenous nucleic acid is DNA or RNA.

14. A method as claimed in any one of claims 10 to 13, wherein the exogenous nucleic acid is an isolated cell nucleus or an isolated spermatozoon.

15. A method as claimed In any one of claims 1 to 14, wherein the fluid of step (b) is a physiologically acceptable fluid selected from physiological saline, an aqueous pH buffered fluid, and a physiologically acceptable polymer.

16. A method as claimed in any one of claims 11 to 15, wherein the avian ovum or avian embryo is obtained from a chicken, turkey, quail, pheasant, duck, goose, ostrich, emu or swan.

17. A method as claimed in claim 16, wherein the avian ovum or avian embryo is obtained from a chicken egg.

18. A method as claimed in any one of claims 10 to 17, wherein the recipient cell in the germinal disk is a blastodermal cell.

19. A method as claimed in any one of claims 11 to 16, wherein an avian ovum or avian embryo is removed from a female bird before hard shell formation.

20. A method as claimed in any one of claims 11 to 19, wherein an avian embryo is delivered to a recipient avian female by fistulation or by delivering to a surgically exposed avian infundibulum.

21. A method for the production of a transgenic bird, which comprises carrying out a method as claimed in any one of claims 10 to 20.

## Patentansprüche

1. Eine Mikroinjektionsanordung für die Einbringung einer exogenen Nukleinsäure in die Keimscheibe eines Vogels, umfassend:
a) ein optisches Mikroskop
b) ein Mikroinjektionssystem; und
c) eine indirekte Makroüberwachungseinheit.

2. Eine Mikroinjektionsanordnung nach Anspruch 1, wobei das Mikroinjektionssystem eine Mikropipette beinhaltet, die funktional mit einem Mikromanipulator und einem Oszillator verbunden ist.

3. Eine Mikroinjektionsanordnung nach Anspruch 2, wobei der Oszillator ein Piezoelektrischer Oszillator ist.

4. Eine Mikroinjektionsanordnung nach einem der Ansprüche 1 bis 3, wobei das optische Mikroskop mit einem Auflichtbeleuchtungssystem ausgestattet ist.

5. Eine Mikroinjektionsanordnung nach Anspruch 4, wobei das optische Mikroskop ein Objektiv mit einer optischen Achse hat, und wobei das Auflichtbeleuchtungssystem entlang der optischen Achse des Objektivs angeordnet ist.

6. Eine Mikroinjektionsanordnung nach einem der Ansprüche 2 bis 5, wobei der Mikromanipulator programmierbar ist.

7. Eine Mikroinjektionsanordnung nach einem der Ansprüche 1 bis 6, wobei die indirekte Überwachungseinheit eine Linse beinhaltet, die funktional mit einer elektronischen Kamera und einer Überwachungseinheit verbunden ist.

8. Eine Mikroinjektionsanordnung nach einem der Ansprüche 1 bis 7, wobei das optische Mikroskop über ein Durchlichtbeleuchtungssystem verfügt.

9. Eine Mikroinjektionsanordnung nach einem der Ansprüche 1 bis 8, wobei das Mikroinjektionssystem und die indirekte Überwachungseinheit an dem optischen Mikroskop befestigt sind.

10. Ein Verfahren für die Einbringung exogener Nukleinsäuren in die Keimscheibe eines Vogels, beinhaltend
a) die Bereitstellung einer Mikroinjektionsanordnung umfassend ein optisches Mikroskop, eine Mikropipette, eine indirekte Makroüberwachungseinheit und die Keimscheibe eines Vogels,
b) das Befüllen der Mikropipette mit einer Flüssigkeit, die eine exogene Nukleinsäure enthält;
c) das Visualisieren der Mikropipette durch die Überwachung der Position der Mikropipette im Verhältnis zu der Keimscheibe eines Vogels durch das makroskopische Überwachungssystem; und
d) das Einbringen der Flüssigkeit, die eine exogene Nukleinsäure enthält, in die Keimscheibe.

11. Ein Verfahren nach Anspruch 10, wobei die Keimscheibe sich in einem Vogelei oder in einem Vogelembryo befindet.

12. Ein Verfahren nach Anspruch 11, zusätzlich umfassend das Einbringen der Keimscheibe in der Form eines Vogelembryos in einen weiblichen Empfängervogel; das Zulassen des Ablegens des Vogelembryos in einem Hartschalenei; und das Zulassen der Entwicklung und des Schlüpfens des Embryos als Kücken.

13. Ein Verfahren nach einem der Ansprüche 10 bis 12, wobei die exogene Nukleinsäure eine isolierte Nukleinsäure ist, ausgewählt aus einem Plasmid, einem viralen Vektor und einer linearen Nukleinsäure, und wobei die exogene Nukleinsäure DNA oder RNA ist.

14. Ein Verfahren nach einem der Ansprüche 10 bis 13, wobei die exogene Nukleinsäure ein isolierter Zellkern oder ein isoliertes Spermatozoon ist.

15. Eine Methode nach einem der Ansprüche 1 bis 14, wobei die Flüssigkeit aus Schritt b) eine physiologisch akzeptable Flüssigkeit ist, ausgewählt aus physiologischer Kochsalzlösung, einer wässrigen pH-gepufferten Flüssigkeit, und einem physiologisch akzeptablen Polymer.

16. Ein Verfahren nach einem der Ansprüche 11 bis 15, wobei das Vogelei oder der Vogelembryo von einem Huhn, einem Truthahn, einer Wachtel, einem Fasan, einer Ente, einer Gans, einem Strauß, einem Emu oder einem Schwan gewonnen wurde.

17. Ein Verfahren nach Anspruch 16, wobei das Vogelei oder der Vogelembryo aus einem Hühnerei gewonnen wurde.

18. Ein Verfahren nach einem der Ansprüche 10 bis 17, wobei die Empfängerzelle in der Keimscheibe eine blastodermale Zelle ist.

19. Ein Verfahren nach einem der Ansprüche 11 bis 16, wobei das Vogelei oder der Vogelembryo aus dem weiblichen Vogel vor der Bildung einer harten Schale entfernt wird.

20. Ein Verfahren nach einem der Ansprüche 11 bis 19, wobei der Vogelembryo in den weiblichen Empfängervogel durch Fistulierung eingebracht wird oder durch Ein führung in das chirurgisch offen gelegte Infundibulum eines Vogels.

21. Ein Verfahren zur Erzeugung eines transgenen Vogels, in dessen Rahmen eines der Verfahren nach den Ansprüchen 10 bis 20 ausgeführt wird.

## Revendications

1. Assemblage de micro-injection pour introduire un acide nucléique exogène dans un disque germinatif aviaire, comprenant :
un microscope optique ;
un système de micro-injection ; et
une unité de macro-surveillance oblique.

2. Assemblage de micro-injection selon la revendication 1, dans lequel le système de micro-injection comprend une micropipette connectée de manière fonctionnelle à un micromanipulateur et un oscillateur.

3. Assemblage de micro-injection selon la revendication 2, dans lequel l'oscillateur est un oscillateur piézo-électrique.

4. Assemblage de micro-injection selon l'une quelconque des revendications 1 à 3, dans lequel le microscope optique possède un système d'éclairage incident.

5. Assemblage de micro-injection selon la revendication 4, dans lequel le microscope optique possède un objectif avec un axe optique, et dans lequel le système d'éclairage incident est dirigé le long de l'axe optique de l'objectif.

6. Assemblage de micro-injection selon l'une quelconque des revendications 2 à 5, dans lequel le micromanipulateur est programmable.

7. Assemblage de micro-injection selon l'une quelconque des revendications 1 à 6, dans lequel l'unité de surveillance oblique comprend une lentille connectée de manière fonctionnelle à une caméra électronique et une unité de surveillance.

8. Assemblage de micro-injection selon l'une quelconque des revendications 1 à 7, dans lequel le microscope optique possède un système d'éclairage par lumière transmise.

9. Assemblage de micro-injection selon l'une quelconque des revendications 1 à 8, dans lequel le système de micro-injection et l'unité de surveillance oblique sont fixés au microscope optique.

10. Procédé d'introduction d'un acide nucléique exogène dans un disque germinatif aviaire, comprenant :
(a) la fourniture d'un assemblage de micro-injection comprenant un microscope optique, une micropipette, un système de macro-surveillance oblique et un disque germinatif aviaire ;
(b) le chargement de la micropipette avec un fluide dans lequel se trouve un acide nucléique exogène ;
(c) la visualisation de la micropipette en surveillant la position de la micropipette par rapport au disque germinatif aviaire grâce au système de macro-surveillance oblique ; et
(d) l'introduction du fluide dans lequel se trouve l'acide nucléique exogène dans le disque germinatif.

11. Procédé selon la revendication 10, dans lequel le disque germinatif est présent dans un ovule aviaire ou un embryon aviaire.

12. Procédé selon la revendication 11, comprenant en outre l'introduction du disque germinatif sous la forme d'un embryon aviaire dans une femelle aviaire receveuse ; le fait de permettre à l'embryon aviaire d'être pondu dans un oeuf à coque dure ; et le fait de permettre à l'embryon de se développer et d'éclore sous la forme d'un poussin.

13. Procédé selon l'une quelconque des revendications 10 à 12, dans lequel l'acide nucléique exogène est un acide nucléique isolé choisi parmi un plasmide, un vecteur viral et un acide nucléique linéaire, et dans lequel l'acide nucléique exogène est un ADN ou un ARN.

14. Procédé selon l'une quelconque des revendications 10 à 13, dans lequel l'acide nucléique exogène est un noyau cellulaire isolé ou un spermatozoïde isolé.

15. Procédé selon l'une quelconque des revendications 1 à 14, dans lequel le fluide de l'étape (b) est un fluide physiologiquement acceptable choisi parmi une solution physiologique saline, un fluide aqueux à pH tamponné et un polymère physiologiquement acceptable.

16. Procédé selon l'une quelconque des revendications 11 à 15, dans lequel l'ovule aviaire ou l'embryon aviaire sont obtenus à partir d'une poule, d'une dinde, d'une caille, d'une faisane, d'une cane, d'une oie, d'une autruche, d'un émeu ou d'un cygne.

17. Procédé selon la revendication 16, dans lequel l'ovule aviaire ou l'embryon aviaire sont obtenus à partir d'un oeuf de poule.

18. Procédé selon l'une quelconque des revendications 10 à 17, dans lequel la cellule receveuse dans le disque germinatif est une cellule blastodermique.

19. Procédé selon l'une quelconque des revendications 11 à 16, dans lequel un ovule aviaire ou un embryon aviaire sont retirés d'un oiseau femelle avant la formation d'une coque dure.

20. Procédé selon l'une quelconque des revendications 11 à 19, dans lequel un embryon aviaire est introduit dans une femelle aviaire receveuse par fistulation ou par introduction dans un infundibulum aviaire chirurgicalement exposé.

21. Procédé de production d'un oiseau transgénique, comprenant la mise en oeuvre d'un procédé selon l'une quelconque des revendications 10 à 20.
